(19) Europäisches Patentamt / European Patent Office / Office européen des brevets

(11) **EP 2 343 552 A1**

(12) **EUROPEAN PATENT APPLICATION**

(43) Date of publication:
13.07.2011 Bulletin 2011/28

(51) Int Cl.:
*G01N 33/543* (2006.01)    *G01N 33/551* (2006.01)
*G01N 33/53* (2006.01)    *G01N 33/536* (2006.01)

(21) Application number: 10012197.9

(22) Date of filing: 08.01.2001

(84) Designated Contracting States:
AT BE CH CY DE DK ES FI FR GB GR IE IT LI LU MC NL PT SE TR
Designated Extension States:
AL LT LV MK RO SI

(30) Priority: 07.01.2000 US 175086 P

(62) Document number(s) of the earlier application(s) in accordance with Art. 76 EPC:
01901846.4 / 1 244 913

(71) Applicant: **Aventis Pasteur**
**Swiftwater, PA 18370-9187 (US)**

(72) Inventor: **Dougherty, Cristy, S.**
**Bath, PA 18014 (US)**

(74) Representative: **Zwicker, Jörk et al**
**Dr. Volker Vossius**
**Patent- und Rechtsanwaltskanzlei**
**Geibelstrasse 6**
**81679 München (DE)**

Remarks:
•This application was filed on 30-09-2010 as a divisional application to the application mentioned under INID code 62.
•Claims filed after the date of filing of the application (Rule 68(4) EPC).

(54) **Competitive enzyme immunoassay for assessing total antigen content of aluminum-adsobed antigens**

(57)    The present invention describes a competitive EIA for assessing total antigen content of aluminum adsorbed antigens. The competitive enzyme immunoassay method of the present invention provides a means for reproducibly quantitating antigens adsorbed to aluminum hydroxide without desorption or pretreatment steps.

Figure 1

## Description

## BACKGROUND OF THE INVENTION

**Field of the Invention**

This invention relates to the field of quantitative determination of antigen content in aluminum-adsorbed antigen vaccine compositions.

**Summary of the Related Art**

Vaccines are comprised of antigens intended to produce a desired protective immune response. Adjuvants have been incorporated in many vaccines to enhance the immune response to various antigens. Some adjuvant types include oil emulsions (Freund's adjuvant), aluminum salts and synthetic compounds (poly[di(carboxylatophenoxy)]phosphazene). Currently, the only adjuvants approved for human use are the aluminum containing adjuvants (1). These are broken down into two types: aluminum hydroxide and aluminum phosphate (3). Aluminum hydroxide adjuvant is crystalline aluminum oxyhydroxide with the mineralogical name of boehmite. Aluminum phosphate adjuvant is amorphous aluminum hydroxyphosphate. Researchers have shown that aluminum hydroxide adjuvant has a high point of zero charge (PZC) and is a good absorbent for acidic proteins, as compared to aluminum phosphate which has a low PZC and thus is better able to absorb more basic proteins (4).

Regulatory authorities require that vaccines be tested to quantitate the antigen content in the final vaccine product. The addition of adjuvants with high absorptivity such as those containing aluminum complicates such quantitation by making the antigen unavailable for detection.

One approach to the quantitation of antigens bound to aluminum is to desorb or elute the alum away from the antigen prior to performing the quantitation assay. For example. phosphate anion has been found to desorb ovalbumin (5), porcine parvovirus (6). and FMDV-antigen (7) from $Al(OH)_3$ adjuvant. In the study by Katz, 66% of total protein could be recovered after desorption of the antigen using phosphate anion. The protein antigens were also tested for activity and was shown to be active.

Another approach to quantitation of antigens adsorbed to aluminum entails elution of the antigens from aluminum-containing adjuvants by treatment with various surfactants (8). Unfortunately, the surfactant most effective for eluting protein antigens from aluminum adjuvants provides only a 50°₀ protein elution (8). Furthermore, surfactants are known to interfere with immunological complexes. Indeed, this interference phenomenon is exploited routinely in immunoassays by incorporating surfactants into washing buffers (9).

A related approach, described by Scattergood and Grabner in U.S. Patent No. 4. 894.444. entails dissolution of the antigen-aluminum complexes by dissolving the aluminum away from the antigen with an alkali metal salt of mono, di or tricarboxylic acid. This method requires careful selection of dissociation conditions, both to ensure complete dissociation of aluminum from the antigen, and to avoid inactivation or denaturation of the antigen of interest. Thus. such a method may not be used with labile antigens. Also, the method involves several steps, including careful adjustment of the pH of the dissociation solution. followed by careful monitoring of the reaction over a period of from 1 to 3 hours, neutralizing the dissociation solution. then recovering the dissociated antigen by dialysis or ultrafiltration.

To date, there has not been a method to reproducibly quantitate antigens adsorbed to aluminum hydroxide without desorption. All of the present methods are quite labor intensive. requiting a lengthy elution times, followed by dialysis and concentration steps. Such methods are unsatisfactory for routine commercial use. Thus. a need exists for improved methods capable of precisely and accurately quantitating antigens that have been adsorbed to aluminum hydroxide.

## SUMMARY OF THE INVENTION

The present invention provides an immunoassay for direct quantitation of antigen within vaccine compositions containing aluminum salts. The method of the present invention provides a simple. accurate and precise way to quantitate antigens in vaccine compositions containing aluminum salts without having to first remove the aluminum from the vaccine composition.

## BRIEF DESCRIPTION OF THE DRAWINGS

Figure 1 displays a graph representing assay specificity determined by comparison of assay controls.

Figure 2 display a graph representing assay selectivity determined by testing various absorbed proteins.

## DETAILED DESCRIPTION OF THE INVENTION

The present invention is a method for directly quantitating antigen within vaccine compositions. wherein the antigen has been adsorbed to aluminum salts. By "direct" is meant that the antigen within the vaccine composition can be quantitated without first removing the aluminum from the antigen. *i.e.*. without pretreatment. elution or dissociation of aluminum salt from the antigen.

The method of the present invention can be applied to any number of potential antigens within vaccine compositions. Such antigens may be prepared by any means. including purification or partial purification from the native state or by recombinant methods. Similarly. antibodies to antigens can be prepared by standard immunological techniques. Such antibodies may be monoclonal, polyclonal, or monospecific polyclonal antibodies, or combinations thereof.

The method of the present invention may be used to quantitate antigens in a vaccine composition containing combinations of one or more antigens. The immunoassay method of the present invention can be used to quantitate individual antigens within such combination vaccines.

Antigen adsorbed to aluminum salt is quantitated according to the invention by a competitive immunoassay procedure. Antigen that has not be adsorbed to aluminum salt is used to coat reaction plates. Antigen that has been adsorbed to an aluminum salt is allowed to react with antibody to the antigen, resulting in the formation of antigen-antibody complexes. Such antigen-antibody complexes are prepared using reference standard antigen as well as vaccine antigen. The antigen-antibody complexes are then added to the reaction plates and allowed to incubate. The reaction plates are then washed to remove unbound antigen-antibody complexes. and the amount of antibody bound to the reaction plates is determined. The amount of antibody bound to the reaction plate is inversely proportional to the amount of antigen present in the antigen-antibody complexes. A standard curve is prepared using the results obtained from the reference standard antigens. and the amount of antigen present in the vaccine composition is determined with reference to the standard curve.

In the immunoassay method of the present invention, reaction vessels are first coated with an antigen of interest. Antigens of interest include any antigens present within vaccine formulations. For example. PspA or PsaA of S. *pneumoniae.* OspA. OspC or DpbA of *B. burgdorferi.* toxoids of diphtheria or tetanus toxin. to name but a few. Reaction vessels could include, by way of example and not limitation, wells within multi-well reaction plates or individual test tubes. The reaction vessels are prepared by adsorbing antigen to each site. using methods well known to those skilled in the art. It is important to note that the antigen that is to be applied to the reaction site is not adsorbed to an aluminum salt.

"Reference" and "test" samples are then prepared. Reference samples are samples in which the quantity of antigen present in the sample is known, whereas test samples are those in which the quantity of antigen in the sample is to be determined. In a preferred embodiment of the present invention, the reference antigen is adsorbed with 520 g of aluminum hydroxide per ml prior to preparing reference samples.

Reference and test samples are prepared at a plurality of dilutions within the range of detection of standard immunoassays (e.g., ng to g range) and added to antibody specific to the antigen. In a preferred embodiment. monoclonal antibody is used. These reaction samples. containing antigen-aluminum and antibody. are transferred to the reaction vessels and incubated for several hours. Incubation results in the formation of complexes of antigen-aluminum plus antibody. Following incubation. the reaction vessels are washed several times to remove unreacted complexes. At this point, the amount of antibody that has bound to the reaction vessel is determined.

The amount of antibody that has bound to the reaction vessel can be determined by any number of methods well known to those skilled in the an. The antibody can be labeled, e.g.. with a radioisotope such as $I^{125}$ or with an enzymatic label. at any point prior to addition of the antibody to the antigen. The antibody could also be labeled with a ligand such as avidin or biotin. and its presence then could be determined indirectly by reaction with labeled anti-ligand (*e.g.*. biotin for avidin. or *vice versa).* Alternatively, the amount of antibody bound to the reaction vessel can readily be determined indirectly by reaction with a labeled secondary antibody, *i.e.* an antibody that is capable of binding specifically to the first antibody. For example, if mouse monoclonal antibodies are used in the reaction sample step, then binding of mouse monoclonal antibodies to the reaction vessels can be determined following a reaction with goat anti-mouse antibody which is labeled, e.g., with a radioisotope, an enzyme, a fluorescent dye or a visible dye.

Some of the antibody in the reaction solution binds to the antigen that was applied to the reaction vessel. Antigen within the reaction solution competes with antigen on the reaction vessel. Therefore. the more antigen there is in the reaction solution. the less antibody will bind to the reaction vessel. Thus, the amount of antibody that binds to the reaction vessel is inversely proportional to the amount of antigen that was present in the reaction solution.

Having determined the amount of antibody that has been bound to each reaction vessel, a standard curve is prepared in which the amount of antibody bound is plotted vs. the amount of antigen present in each reference sample. The results obtained with dilutions of the test sample are compared to this standard curve, and the amount of antigen present within the test sample is readily determined with reference to the standard curve.

The following non-limiting examples further illustrate the present invention.

**EXAMPLES**

Reagents and Conditions:

Chemicals: Bovine serum albumin (BSA). alkaline phosphatase substrate (ABTS), and TRIZMA base were from Sigma (St. Louis. MI. USA). Al(OH) gel (alhydrogel) was from Superfos (Vedbaek. Denmark). Tween-20 was from BioRad (Hercules, CA. USA). $NaHCO_3$. $Na_2CO_3$, and $M_gCl_2 \bullet 6H_2O$ were from JTBaker (Phillipsburg, NJ. USA). NaCl. $Na_.HPO_4$. $KH_2PO_4$. NaOH were from VWR (Pittsburg, PA, USA).

Antibodies: Monoclonal antibodies were made by immunizing Balb/c female mice three times intraperitoneally at approximately one month intervals and one time intravenously three days prior to spleen harvesting. Spleen cells were fused using the PEG cell fusion method described by Lane (11) to the murine myeloma cell line P3X63Ag8.653 (ATCC CRL1580). Hybridoma cells were selected using HAT media (hypoxanthine, aminopterin, and thymidine) and screened by ELISA for IgG antibodies to the specific protein of interest. Alkaline phosphatase conjugated goat anti-mouse IgG antibodies were from Cappel (ICN. Aurora. Ohio. USA).

Buffers: Filters ($0.22\mu m$ pore size) and Milli Q plus water purification system for purified water were from Millipore (Boston, MA, USA). Phosphate buffered saline (PBS) at 0.01 M phosphate and 0.85% NaCl with a pH 7.2 $\pm$ 0.1 was prepared by the PMC Bioservices Department. Coating buffer for the immunoassay was prepared from $NaHCO_3$ and $Na_2CO_3$ to make a final concentration of 0.015 M $NaCO_3$ and 0.035 M $NaHCO_3$ at pH 9.6 $\pm$ 0.1. Blocking buffer was 2.0% BSA in PBS. Washing buffer was 0.05% Tween-20 in PBS. Diluent used for sample dilutions was 1.0% BSA in PBS. Alkaline phosphatase substrate buffer was 0.05M $NaCO_3$. 1.0 mM $MgCl_2$ at pH of 9.8 $\pm$ 0.1. Enzymatic stopping solution is 5 N NaOH. TRIS buffered saline (TBS) buffers at 1.0 M and 25 mM TRIS with 0.85% NaCl at pH 7.0 = 0.1 were prepared by the PMC Product Development Department.

Antigens: Various strains of recombinant pneumococcal surface protein, proteins (1.2. 3. 4. 5), were prepared by the PMC Product Development Department. These antigens were prepared under GLP/GMP conditions. The recombinant protein was absorbed to 520 $\mu g$ of aluminum/mL of 25mM TBS by batch incubation. The aluminum was in the form of aluminum hydroxide (alhyrdogel. Supertos™).

**Example 1**

*Competitive EIA Procedure*

Following a four plate randomization scheme, reference and vaccine test samples were prepared and tested. Initially, four Immulon IV assay plates (Dynatech, Chantilly, VA, USA) were coated with non-adsorbed PspA antigen diluted in coating buffer by adding 100 $\mu L$ of the diluted antigen to each well of the plate. The assay coated plates were then sealed with a plate sealer (Dynatech. Chantilly, VA. USA) and were incubated for 16-24 hr at 4○C. Simultaneously, an assay dilution plate was prepared. Various concentrations of the reference and vaccine test samples were prepared. The reference, recombinant protein also absorbed to 520 $\mu g$ of aluminum/mL of 25 mM TBS, was diluted to an initial concentration with assay diluent and serially diluted 2-fold down the column of the dilution plate. The adsorbed vaccine test sample was also diluted to the same initial concentration as the reference material and serially diluted 2-fold down the column of the dilution plate. Both the reference and the vaccine test sample were prepared in duplicate on each of the four assay dilution plates. The diluted reference and vaccine test samples were incubated overnight at 4°C with an equal volume of an appropriately diluted, antigen specific. monoclonal antibody. Positive control consisted of the appropriately diluted. antigen specific. monoclonal antibody only. The negative control (or background control) consisted of assay diluent only. Both were incubated in replicates at a single concentration in the assay dilution plate. The next day. the assay coated plates were removed from 4°C and washed six times with washing buffer. Then. the reference/monoctonal mixtures and the vaccine test sample/monoclonal mixtures were transferred from the assay dilution plate to the washed assay coated plate. This was repeated four times. corresponding to each set of the four plates due to the randomization scheme. The assay coated plates were then sealed and incubated for 2 hr at 37 $\pm$ 2°C. The plates were washed six times with washing buffer and then incubated with appropriately diluted alkaline phosphatase conjugated goat anti-mouse 1gG for 1.5 hr at 37 $\pm$ 2°C. The alkaline phosphatase conjugated goat anti-mouse IgG was diluted to an appropriate concentration with assay diluent. After which, the plates were washed with a series of washes one final time. Next. an appropriate concentration of alkaline phosphatase substrate (p-Nitrophenyl phosphate or p-NPP) diluted with substrate buffer was added to all of the wells. The enzymatic reaction was stopped after 60-90 min with 10 $\mu L$ of 5N NaOH and the bound specific monoclonal was detected by monitoring the resulting enzyme product at 405 nm. Absorbance measurements were performed on a SpectraMax™ microtiter plate reader (Molecular Devices. Menlo Park. CA. USA). The antibody bound to the assay coated plate is inversely proportional to the concentration of antigen present in the reference or the vaccine test sample.

**Example 2**

*Quantitation of PspA Adsorbed to Aluminium Hydroxide*

Recombinant pneumococcal surface protein (PspA) was expressed and adsorbed to aluminum hydroxide. The protein was formulated with 520 μg mL aluminum hydroxide to 10. 20, 50, and 100 μ/mL total protein. as detected by Lowry. These samples are referred to as vaccine test samples from this point forth. The vaccine test samples were tested for antigen content by competitive EIA using an aluminum hydroxide adsorbed reference material. The reference and vaccine test samples were diluted to the same initial starting concentration of 1.0 μg/mL. The assays were performed as previously described in Example 1. To assess the assay for precision, accuracy, and parallelism, a series of four independent runs using a four plate randomization scheme was performed. The antigenic content was calculated for the four vaccine test samples by interpolating a concentration from the reference and then correcting for dilution. The calculated antigenic content was then statistically analyzed for precision. accuracy, and parallelism.

Precision is defined as the closeness of agreement, or the degree of scatter, among a series of measurements obtained from multiple sampling of the same homogeneous sample under prescribed conditions. In the analysis performed, precision was calculated by transforming the data into the natural log and using equation 1.

$$\text{Equation 1: Precision} = (e^{\sqrt{\sigma_b^2 + \sigma_w^2}} - 1) \times 100\%$$

Accuracy was also assessed and reported as percent recovery. This is defined as the average of the interpolated, dilution-corrected concentrations of the vaccine test sample relative to that of the total protein value determined by Lowry. The percent recovery is calculated by equation 2 below:

$$\text{Equation 2: } \% \text{ Recovery} = 100\% \times (\text{Calculated antigen content/Lowry value})$$

Lastly. parallelism was determined using the dilution effect equation depicted by equation 3:

$$\text{Equation 3: Dilution Effect} = 100 \times 2^{1-(b_T/b_S)} - 1 \text{,}$$

where $b$, is the slope of the test sample and $b_s$ is the slope of the reference.

The percent dilution effect provides an indication of parallelism between the dilution series of the reference as it compares to the dilution series of the vaccine test sample(s). The effect of dilution can be estimated as the percent deviation at 2-fold dilution of the test article. using the slopes of the reference and the vaccine test sample profiles. The assay which exhibits perfect parallelism has a dilution effect value of 0%.

Table I represents the statistical summary of the calculated antigen concentration values obtained for the four vaccine test samples tested.

**Table 1**

| Summary of results from 4 independent runs using the four plate randomization scheme with 8 dilution points for the reference and each vaccine test sample, n=128 | | | | |
|---|---|---|---|---|
| **Concentration of Vaccine Test Sample** | **Average Antigen Concentration (μg/mL)** | **% Relative Standard Deviation (Precision)** | **% Recovery (Accuracy)** | **% Dilution Effect** |
| 10 μg/mL | 8.72 ± 1.44 | 17.9 | 87.2 | -0.443 |
| 20 μgmL | 18.47 ± 3.36 | 19.7 | 92.4 | -0.711 |

(continued)

| Summary of results from 4 independent runs using the four plate randomization scheme with 8 dilution points for the reference and each vaccine test sample, n=128 | | | | |
|---|---|---|---|---|
| Concentration of Vaccine Test Sample | Average Antigen Concentration ($\mu$g/mL) | % Relative Standard Deviation (Precision) | % Recovery (Accuracy) | % Dilution Effect |
| 50 $\mu$g/mL | 51.35 $\pm$ 7.54 | 15.9 | 103 | -0.201 |
| 104 $\mu$g/mL | 96.4 $\pm$ 11.6 | 13.3 | 95.5 | 0.815 |

Precision:

Precision was assessed for the 10. 20. 50. and 100 $\mu$g mL formulated vaccine test samples over different days with different assay buffers. Table I above lists the average precision value as the percent relative standard deviation and shows that the % RSD obtained over the different days ranged from 13.3 to 19.7%. However, table 2 further defines the precision for each day with precision values range from 6.35 - 20.9%.

**Table 2**

| Precision values obtained over four different days. n=32 | | | | |
|---|---|---|---|---|
| Concentration of vaccine Test Sample | % Relative Standard Deviation Day 1 | % Relative Standard Deviation Day 2 | % Relative Standard Deviation Day 3 | % Relative Standard Deviation Day 4 |
| 10 $\mu$g/mL | 15.1 | 14.5 | 18.5 | 18.2 |
| 20 $\mu$g/mL | 20.9 | 18.9 | 18.2 | 18.9 |
| 50 $\mu$g/mL | 13.8 | 16.9 | 15.7 | 12.7 |
| 100 $\mu$g/mL | 7.53 | 14.6 | 7.93 | 6.35 |

Accuracy:

Results generated by the competitive E1A method were compared to dilution corrected Lowry values. Since the material being tested is adsorbed to aluminum hydroxide, it is difficult to measure total protein content after protein absorption. Also, the Lowry is providing a total protein concentration value which is not specific to the antigen in question. However. the dilution corrected Lowry values of the pre-adsorbed material is the only value available for comparison at the time of assay design. Table 3 summarizes the accuracy values obtain for each of the days independently and as an overall measure.

**Table 3**

| Accuracy values obtained during testing | | | | |
|---|---|---|---|---|
| Concentration of Vaccine Test Sample | % Recovery Day 1 n=32 | % Recovery Day 2n=32 | % Recovery Day 3 n=32 | % Recovery Day 4 n=32 | % Recovery Overall n=128 |
| 10 $\mu$g/mL | 93.6 | 80.7 | 82.5 | 91.4 | 87.2 |
| 20$\mu$g/mL | 97.3 | 87.1 | 89.0 | 95.9 | 92.4 |
| 50 $\mu$g/mL | 107 | 95.0 | 100 | 109 | 103 |
| 100 $\mu$g/mL | 103 | 87.1 | 86.8 | 106 | 86.8 |

The accuracy values range from 80.7 - 109% and are consider acceptable for an immunological procedure.

Parallelism:

To assess parallelism, the log of the OD values are plotted against the log of the protein concentration. The slopes of the reference and the vaccine test samples are compared for percent bias using equation 3 above. Table 4 contains the percent dilution effect obtained for each run and over all the runs.

**Table 4**

| Percent Dilution Effect (percent bias) for each vaccine test sample as a measure of parallelism. | | | | | |
|---|---|---|---|---|---|
| **Concentration of vaccine Test Sample** | **% Bias Day 1 n=32** | **% Bias Day 2 n=32** | **% Bias Day 3 n=32** | **% Bias Day 4 n=32** | **% Bias Overall n=128** |
| 10 μg/mL | -0.72 | 0.39 | -1.34 | -1.07 | -0.44 |
| 20 μg/mL | -0.43 | 0.65 | -1.38 | 0.34 | -0.71 |
| 50 μg/mL | -1.47 | 0.68 | -2.60 | -1.47 | -0.20 |
| 100 μg/mL | 0.21 | 2.93 | -0.87 | 0.98 | 0.82 |

Since 0% dilution effect represents perfect parallelism, the parallelism results shown in table 4 are excellent, ranging from -2.60 to 2.93 % bias over a concentration range of 0.008 - 1.0 μg/mL.

Speciticity/Setectivity:

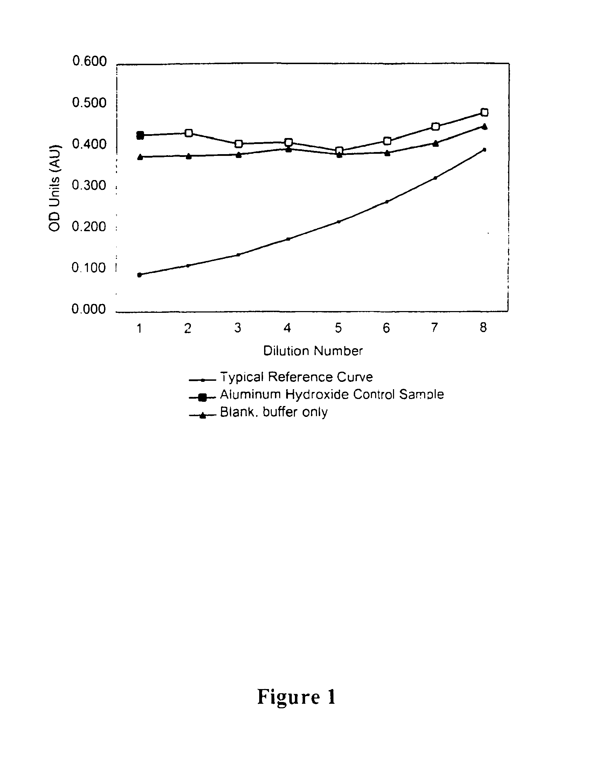

To determine if the aluminum hydroxide interferes in the assay. a variety of experiments were performed. One experiment tested the nonspecific binding of the monoclonal antibody to aluminum hydroxide during the incubation step. Figure 1 depicts the results. There is no difference between the 'blank' samples, buffer only, and the 'control' samples, those containing aluminum hydroxide without adsorbed protein.

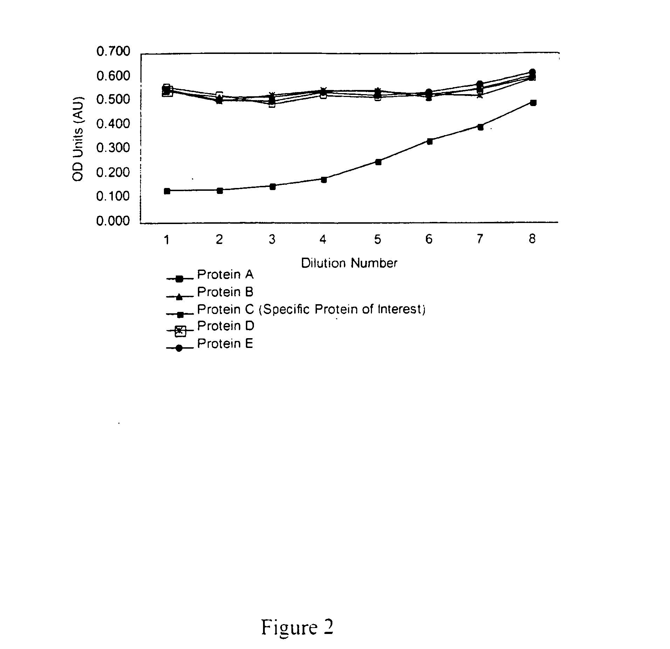

In a separate experiment, various proteins were adsorbed to the aluminum hydroxide and tested in the assay. Only the vaccine test sample corresponding the specific monoclonal in the assay produced a positive response (figure 2). All others produced unchanging OD values independent of dilution, exhibiting a similar profile to the blank depicted in figure 1.

A third experiment was performed to determine the effect of aluminum hydroxide concentration on the calculated antigenicity values. There are no apparent differences in the competitive EIA calculated values for samples containing different amounts of aluminum hydroxide but a constant amount of protein.

Repeatability:

Not only are the conditions repeatable for different operators on different days with different instruments but two other independent antigens have also produced similar precision. accuracy. linearity, and specificity results using the same concept described here.

REFERENCES

1. Edelman, R. Vaccine adjuvants. Rev. lnfect. Dis. 1980. 2, 370-83.

2. World Health Organization. Manual for the Production and Control of Vaccines-Tetanus Toxoid. BLG/UNDP/77.2. Rev. 1. WHO. Geneva. Switerland. 1977.

3. Shirodkar. S, Hutchinson, RL. Perry, DL. White. JL. and Hem. SL. Aluminum compounds used as adjuvants in vaccines. Pharm. Res. 1990. 7. 1282-88.

4. Al-Shakhshir. R. Regnier, F. White. JL. and Hem, SL. Effect of protein adsorption on the surface charge characteristics of aluminum-containing adjuvants. Vaccine. 1994, 12. 473-74.

5. Rinella, JV, Jr., White, JL, and Hem. SL. Effects of anions on model aluminum adjuvant containing vaccines. J. Colloid Interface Sci. 1995. 172, 121-30.

6. Katz, J. Desorption of porcine parvovirus from aluminum hydroxide adjuvant with subsequent viral immunoassay or hemagglutination assay. Veterinary Research Communications. 1987, 11, 83-92.

7. Rinella, JV, Workman, RF. Hermodson. MA. White. JL. and Hem, SL. Elutability of proteins from aluminum-containing vaccine adjuvants by treatment with surfactants. J. Colloid Interface Sci. 1998, 197. 48-56.

8. Visser. N. and Woortmeyer. R. Quantification, characterization and safetytesting of foot-and-mouth disease virus antigens eluted from alhydrogel vaccines. Dev. Biol. Stand. 1981. 50. 277-83.

9. Harlow, E and Lane, D. Using Antibodies: A Laboratory Manual. Cold Spring Harbor Laboratory Press. Cold Spring Harbor, NY. 1999.

10. Houen, G and Koch. C. A non-denaturing enzyme linked immunosorbent assay with protein preadsorbed onto aluminum hydroxide. J Immunol. Methods. 1997. 200. 99-105.

11. Lane. R.D. A short-duration polyethylene glycol fusion technique for increasing production of monoclonal antibody-secreting hybridomas. J Immunol. Methods. 1985. 81. 223-228.

1. A competitive immunoassay method for quantitating antigen in a vaccine composition containing aluminum salts, the method comprising:

a) binding antigen that has not been adsorbed to an aluminum salt to a plurality of identical reaction vessels;

b) adsorbing a known quantity of reference antigen to an aluminum salt to form a reference standard:

c) preparing dilutions of test samples and reference standard of antigen in the presence of antibody to the antigen to form a series of individual reaction mixtures:

d) adding each reaction mixture to an individual reaction vessel and incubating for a period of several hours to allow for the formation of antigen-antibody complexes:

e) removing reaction mixtures from the reaction vessels and washing the reaction vessels to eliminate antigen-antibody complexes in solution: and

f) determining the amount of antibody which has bound to each reaction vessel.

2. The method of item 1, wherein the aluminum salt is aluminum hydroxide.

3. The method of item 1, wherein the antigen is PspA.

4. The method of item 1. wherein the antibody is monoclonal antibody to PspA.

## Claims

1. A competitive immunoassay method for quantitating antigen in a vaccine composition containing aluminum salts, the method comprising:

a) binding antigen that has not been adsorbed to an aluminum salt to a plurality of identical reaction vessels;
b) adsorbing a known quantity of reference antigen to an aluminum salt to form a reference standard;
c) preparing dilutions of test samples and reference standard of antigen in the presence of antibody to the antigen to form a series of individual reaction mixtures;
d) adding each reaction mixture to an individual reaction vessel and incubating for a period of several hours to allow for the formation of antigen-antibody complexes;
e) removing reaction mixtures from the reaction vessels and washing the reaction vessels to eliminate antigen-antibody complexes in solution; and

   f) determining the amount of antibody which has bound to each reaction vessel.

**2.** The method of claim 1. wherein the aluminum salt is aluminum hydroxide.

**3.** The method of claim 1, wherein the antigen is PspA.

**4.** The method of claim 1. wherein the antibody is monoclonal antibody to PspA.

**Figure 1**

Figure 2

Europäisches
Patentamt

European
Patent Office

Office européen
des brevets

## EUROPEAN SEARCH REPORT

Application Number

EP 10 01 2197

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 199326<br>Thomson Scientific, London, GB;<br>AN 1993-212602<br>XP002170319,<br>& SU 1 746 318 A1 (POLIOMYELITIS VIRUS INST) 7 July 1992 (1992-07-07)<br>* abstract * | 1-4 | INV.<br>G01N33/543<br>G01N33/551<br>G01N33/53<br>G01N33/536 |
| X | KATZ J B ET AL: "IN-VITRO ASSESSMENT OF VIRAL ANTIGEN CONTENT IN INACTIVATED ALUMINUM HYDROXIDE ADJUVANTED VACCINES",<br>JOURNAL OF VIROLOGICAL METHODS,<br>vol. 25, no. 1, 1989, pages 101-108,<br>XP001009902,<br>ISSN: 0166-0934<br>* the whole document * | 1-4 | |
| X | JENSEN O M ET AL: "ON THE EFFECT OF ALUMINUM HYDROXIDE AS AN IMMUNOLOGICAL ADJUVANT",<br>APMIS (ACTA PATHOLOGICA MICROBIOLOGICA ET IMMUNOLOGICA SCANDINAVICA),<br>vol. 96, no. 3, 1988, pages 257-264,<br>XP001009909,<br>ISSN: 0903-4641<br>* page 258, column 2, paragraph 2 * | 1-4 | TECHNICAL FIELDS SEARCHED (IPC)<br><br>G01N |
| A | EP 0 589 348 A (BEHRINGWERKE AG)<br>30 March 1994 (1994-03-30)<br>* the whole document * | 1-4 | |

The present search report has been drawn up for all claims

| Place of search | Date of completion of the search | Examiner |
|---|---|---|
| Munich | 11 May 2011 | Luis Alves, Dulce |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 01 2197

11-05-2011

| Patent document cited in search report | | Publication date | Patent family member(s) | | Publication date |
|---|---|---|---|---|---|
| SU 1746318 | A1 | 07-07-1992 | NONE | | |
| EP 0589348 | A | 30-03-1994 | AT | 287538 T | 15-02-2005 |
| | | | AU | 4754993 A | 31-03-1994 |
| | | | CA | 2106914 A1 | 26-03-1994 |
| | | | DE | 4232073 A1 | 31-03-1994 |
| | | | DE | 59310372 D1 | 24-02-2005 |
| | | | DK | 0589348 T3 | 30-05-2005 |
| | | | ES | 2236683 T3 | 16-07-2005 |
| | | | JP | 6265549 A | 22-09-1994 |
| | | | PT | 589348 E | 31-05-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4894444 A

**Non-patent literature cited in the description**

- **EDELMAN, R.** *Vaccine adjuvants. Rev. Infect. Dis.,* 1980, vol. 2, 370-83
- Manual for the Production and Control of Vaccines-Tetanus Toxoid. BLG/UNDP/77.2. Rev. 1. WHO. World Health Organization, 1977
- **SHIRODKAR. S ; HUTCHINSON, RL. ; PERRY, DL. ; WHITE. JL. ; HEM. SL.** Aluminum compounds used as adjuvants in vaccines. *Pharm. Res.,* 1990, vol. 7, 1282-88
- **AL-SHAKHSHIR. R. ; REGNIER, F. ; WHITE. JL. ; HEM, SL.** Effect of protein adsorption on the surface charge characteristics of aluminum-containing adjuvants. *Vaccine,* 1994, vol. 12, 473-74
- **RINELLA, JV, JR. ; WHITE, JL ; HEM. SL.** Effects of anions on model aluminum adjuvant containing vaccines. *J. Colloid Interface Sci.,* 1995, vol. 172, 121-30
- **KATZ, J.** Desorption of porcine parvovirus from aluminum hydroxide adjuvant with subsequent viral immunoassay or hemagglutination assay. *Veterinary Research Communications,* 1987, vol. 11, 83-92
- **RINELLA, JV ; WORKMAN, RF. ; HERMODSON. MA. ; WHITE. JL. ; HEM, SL.** Elutability of proteins from aluminum-containing vaccine adjuvants by treatment with surfactants. *J. Colloid Interface Sci.,* 1998, vol. 197, 48-56
- **VISSER. N. ; WOORTMEYER. R.** Quantification, characterization and safetytesting of foot-and-mouth disease virus antigens eluted from alhydrogel vaccines. *Dev. Biol. Stand.,* 1981, vol. 50, 277-83
- **HARLOW, E ; LANE, D.** Using Antibodies: A Laboratory Manual. Cold Spring Harbor Laboratory Press, 1999
- **HOUEN, G ; KOCH. C.** A non-denaturing enzyme linked immunosorbent assay with protein preadsorbed onto aluminum hydroxide. *J Immunol. Methods,* 1997, vol. 200, 99-105
- **LANE. R.D.** A short-duration polyethylene glycol fusion technique for increasing production of monoclonal antibody-secreting hybridomas. *J Immunol. Methods,* 1985, vol. 81, 223-228